Europäisches Patentamt

European Patent Office

Office européen des brevets

(11) Publication number: 0 378 411
A1

# EUROPEAN PATENT APPLICATION

(21) Application number: 90300317.6

(22) Date of filing: 11.01.90

(51) Int. Cl.5: A61K 7/00

(30) Priority: 11.01.89 GB 8900549

(43) Date of publication of application:
18.07.90 Bulletin 90/29

(84) Designated Contracting States:
AT BE CH DE DK ES FR GR IT LI LU NL SE

(71) Applicant: DELRIMS LIMITED
1 Hickmans Road Birkenhead
Wirral L41 1JH(GB)

(72) Inventor: Stackhouse, Alan Linford
20 Bebington Road Higher Tranmere
Birkenhead Merseyside, L42 6PU(GB)

(74) Representative: Lyons, Andrew John et al
ROYSTONS 531 Tower Building Water Street
Liverpool L3 1BA(GB)

(54) Laminates.

(57) A perfume sampler comprises first and second layers pedable apart. The first layer at least carries microencapsulated particles containing a fragrance. Peeling the two layers apart exposes the microencapsulated particles whereupon pressure cause them to rupture and release their fragrance.

EP 0 378 411 A1

## Laminates

This invention concerns laminates and, in particular, concerns laminates for holding perfumes/fragrances for sampling purposes.

It is known to impregnate or coat materials, such as cardboard and paper, with fragrances or perfumes for sampling purposes. It is further known to produce perfume samplers by coating paper leaflets with a slurry of microencapsulated particles containing a perfume/fragrance, the leaflets normally will be folded over so that when opened the microencapsulated particles are ruptured to release the perfume/fragrance. The opened sampler is usually rubbed onto the skin for the perfume/fragrance to be sampled properly.

Such samples are not particularly effective, since very small micro-capsules have to be used and often insufficient perfume/fragrance is released for a useful sample. Larger micro-capsules are not really usable because they are vulnerable to premature damage due to pressure and handling stress on the leaflets. In addition, such premature release can impair the quality of the supporting paper and any printing thereon or on any leaflet, magazine or the like with which the sampler is provided.

An object of this invention is to provide a sampler for perfumes/fragrances that is less susceptible to the above disadvantages than hitherto available samplers.

According to the present invention there is provided a laminar structure having microencapsulated particles biassed to at least a first layer and a second layer, said layers being separable to reveal the microencapsulated particles.

The first layer of the laminar structure is preferably of a flexible sheet material. The flexible sheet material is preferably porous, whereby the microencapsulated can adhere thereto. Preferably also the flexible sheet material has a relief or denticulated surface pattern or texture to provide a cushioning effect so that micro-capsules in or on the first layer are less likely to be damaged in the laminar structure. Other desirable properties of the flexible sheet material include adequate tensile strength typically M/C 350, XM/C (N/5cm), tear strength typically M/C 27, XM/C 42 (N) and burst strength typically about 635 Mullen KN/M$^2$. The weight and thickness of the flexible sheet material may also be important. Typically the flexible sheet material will have a thickness of from 0.20 to 0.40 mm, especially about 0.25 mm. The weight of the flexible sheet material will preferably be in the range of 50 to 120 g/m$^2$, especially about 80 g/m$^2$.

Finally, the flexible sheet material will preferably be printable, for which the texture of the sheet material may be important. Printability is desirable so that patterns, designs or other graphic or pictorial information may be printed on the laminate.

A suitable flexible sheet material for use in the laminate of the invention is a sheet material formed from non-woven polyester fibres.

Both first and second layers of the laminate of the invention may be made from the same material. It is important, however, that the two layers be readily separable or peelable.

According to the present invention there is further provided a method of producing a laminar structure comprising a first layer having microencapsulated particles biassed thereto and a second layer, the layers being separable, comprising the steps of coating a first layer with a slurry of microencapsulated particles and an adhesive substance, coating a second layer with a release agent on its surface to contact the coated surface of the first layer, bringing the two layers together and drying same.

The first layer of the laminar structure of the invention may be produced by coating one side of a sheet of flexible sheet material with an aqueous slurry of microencapsulated particles. The slurry preferably also includes an adhesive material. Typically a slurry for use in the invention will comprise water, microencapsulated particles, an adhesive substance such as a natural gum or synthetic polymer adhesive substance and gelatine. A suitable gum is gum acacia.

The microencapsulated particules are preferably of a size in the range of 250 to 400 $\mu$ and preferably have a cell wall to liquid fill volume ratio of about 3:7.

The second layer of the laminar structure of the invention preferably has a barrier coating on its surface to contact the first layer. Such a barrier coating may be provided by a suitable release agent, of which an example is silicone.

The two layers are preferably brought together when the first layer is freshly coated with the slurry and allowed to dry naturally or with heat assistance, such as on a heated conveyor.

The second layer may also be impregnated or coated with the slurry so that the microencapsulated particles are present in or on both layers of the laminate.

A preferred use of the present invention is in the field of perfume samplers. Thus, the microencapsulated particles will contain a fragrance. When the second layer is peeled from the first layer, the micro-capsules are exposed and pressure thereon will cause them to rupture to release their fragrance. The first layer can then be rubbed onto the skin for proper sampling of the perfume.

A laminate of the invention may have a single fragrance only contained therein either all over or in one or more discrete areas. Alternatively two or more fragrances/perfumes may be contained in the same laminate in discrete areas for separate sampling. The coating of the slurry may be in conjunction with a pattern or design so that the area of the laminate containing a or the perfume to be sampled can be identified.

The thickness of the flexible sheet material of the invention need only be of the 0.20 to 0.40 mm preferably 0.25mm so that a laminate of the invention may only be from 0.40 to 0.80 mm in thickness making it ideal for inclusion as in a magazine or on a display card or the like as a perfume sampler for advertising purposes.

The invention will now be further described by means of the following Example.

Example

An aqueous slurry was prepared by mixing water, microencapsulated fragrance particles (wall to fill volume ratio of 3:7 and particle size 250 to 400 $\mu$), acacia gum and gelatine. The aqueous slurry was evenly coated onto one side of a layer of non-woven polyester fibre sheet material.

A second layer of non-woven polyester fibre sheet material was coated on one side with silicone solution.

The two layers were then brought together with their coated sides facing and dried on a heated conveyor.

The micro-capsules are, thus, supported in a medium which forms a union between the two layers and which hardens the substrate layer sufficiently but not excessively so that the micro-capsules are cushioned and protected against pressures and stress that may otherwise cause them to rupture. The medium is such that the two layers are readily separated by peeling after drying.

Claims

1. A laminar structure having microencapsulated particles biassed to at least a first layer and a second layer, said layers being separable to reveal the microencapsulated particles.

2. A structure as claimed in claim 1, wherein the first layer is of flexible, preferably porous sheet material.

3. A structure as claimed in claims 2 or 3, wherein the flexible sheet material has a relief or denticulated surface pattern or texture.

4. A structure as claimed in claim 1, 2 or 3, wherein the first layer is formed from non-woven polyester fibres.

5. A structure as claimed in anyone of claims 1 to 4, wherein the first and second layers are of the same material.

6. A structure as claimed in any one of claims 1 to 5, wherein the microencapsulated particles are of a size in the range of 250 to 400$\mu$.

7. A structure as claimed in any one of claims 1 to 6, wherein the microencapsulated particles have a cell wall to liquid fill volume ratio of about 3:7.

8. A structure as claimed in any one of claims 1 to 7, wherein the microencapsulated particles contain a fragrance.

9. A method of producing a laminar structure comprising a first layer having microencapsulated particles biassed thereto and a second layer, the layers being separable, comprising the steps of coating a first layer with a slurry of microencapsulated particles and an adhesive substance, coating a second layer with a release agent on its surface to contact the coated surface of the first layer, bringing the two layers together and drying same.

10. A method as claimed in claim 9, wherein the first layer is produced by coating one side of a sheet of flexible sheet material with an aqueous slurry of microencapsulated particles.

11. A method as claimed in claim 9 or 10 wherein the two layers are brought together when the first layer is freshly coated with slurry.

12. A method as claimed in claim 9, 10 or 11, wherein the laminar structure is dried on a heated conveyor.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | EP-A-0 247 864 (QMAX) <br> * Page 3, lines 14-25; page 5, lines 6-24 * | 1,2,5,6 ,7,8,10 ,11 | A 61 K 7/00 |
| X | EP-A-0 189 656 (MINNESOTA MINING AND MANUFACTURING CO.) <br> * Page 7, lines 24-33; page13, lines 25-36 * | 1,2,8 | |
| X | EP-A-0 188 883 (MINNESOTA MINING AND MANUFACTURING CO.) <br> * Page 12, lines 34-36; page 13, lines 1-21 * | 1,2,8, 12 | |
| X | EP-A-0 268 316 (TEN KLEI SPECIALS) <br> * Column 2, lines 26-54; column 3, lines 1-9 * | 1,2,8,9 ,11,12 | |
| A | US-A-4 654 256 (R.H. DOREE et al.) <br> * Column 5, lines 36-46 * | 1,9 | |
| A | DE-A-2 055 019 (THE NATIONAL CASH REGISTER CO.) <br> * Page 3, paragraph 7; page 4, paragraph 4 * | 1,3 | TECHNICAL FIELDS SEARCHED (Int. Cl.5) <br><br> B 01 J |
| A | US-A-3 494 505 (D.K. HUEBNER et al.) <br> * Column 2, lines 25-71 * | 1,4 | |

The present search report has been drawn up for all claims

| Place of search | Date of completion of the search | Examiner |
|---|---|---|
| THE HAGUE | 26-03-1990 | KERRES P.M.G. |

EPO FORM 1503 03.82 (P0401)